# EUROPÄISCHE PATENTANMELDUNG

(11) **EP 1 816 363 A2**
(43) Veröffentlichungstag der Anmeldung: **08.08.2007**
(21) Anmeldenummer: 07101586.1
(22) Anmeldetag: 01.02.2007
(51) Int. Cl.: F16D 1/02, A61B 17/02, A61B 17/56

(54) **KOPPLUNGSVORRICHTUNG**

(30) Priorität: 03.02.2006 EP 06101256
(71) Anmelder: Zimmer GmbH, 8404 Winterthur (CH)
(72) Erfinder: Reinmuth, Jochen, 8406, Winterthur (CH); Filippi, Michael, 8200, Schaffhausen (CH)

(57) **Zusammenfassung**

Kopplungselement, z.B. für ein Distraktionsinstrument für Bandscheibenoperationen, umfassend wenigstens ein Grundelement (11) mit einer Längsachse (13), einer Zentriereinrichtung (15), einer Stirnseite (17) und einer an der Stirnseite (17) ausgebildeten Drehmomentaufnahmeeinrichtung zur Aufnahme eines von einem Gegenelement (51,51') auf das Grundelement (11) bezüglich der Längsachse (13) ausgeübten Drehmomentes, wobei die Drehmomentaufnahmeeinrichtung eine Mehrzahl von ausgehend von der Stirnseite (17) ausgebildeten, in Achsumlaufrichtung mit Abstand voneinander verteilt angeordneten Auflagerelementen (19) umfasst, wobei die Auflagerelemente (19) in einer Ansicht auf die Stirnseite (17) jeweils eine Längsseite (21) und eine Schmalseite (23) aufweisen, wobei die Längsseite (21) in ihrem Verlauf eine größere Länge aufweist als die Schmalseite (23), und wobei die Schmalseite (23) eine sich parallel zur Längsachse (13) und im Wesentlichen senkrecht zur Achsumlaufrichtung erstreckende Auflagerfläche (25) begrenzt, die zur Aufnahme eines in einem ersten Drehsinn wirksamen Drehmomentes von dem Gegenelement (51,51') beaufschlagbar ist.

## Beschreibung

Die Erfindung betrifft allgemein die Kopplung von Elementen, insbesondere von Elementen oder Komponenten, bei denen es sich um Bestandteile von Instrumentensystemen zur Durchführung von Operationen handelt.

An die Kopplung von Elementen werden je nach Anwendung unterschiedliche Anforderungen gestellt. Eine Anforderung, die beispielsweise für Instrumente relevant ist, wie sie bei bestimmten Bandscheibenoperationen eingesetzt werden, besteht darin, relativ hohe Drehmomente, die zwischen den gekoppelten Elementen wirksam sind, übertragen oder aufnehmen zu können, ohne dabei andere geforderte Eigenschaften der Kopplung oder der Elemente zu beeinträchtigen.

Diesen und anderen Anforderungen wird die Erfindung gerecht. Die Erfindung betrifft ein Kopplungselement, das wenigstens ein Grundelement umfasst. Ferner betrifft die Erfindung ein Gegenelement zur Verwendung mit dem Kopplungselement. Die Erfindung betrifft außerdem ein Kopplungssystem umfassend wenigstens ein Grundelement und ein Gegenelement. Des Weiteren betrifft die Erfindung ein Instrumentensystem, insbesondere für Bandscheibenoperationen, das wenigstens ein Manipulationsinstrument und zumindest einen mit dem Manipulationsinstrument koppelbaren Funktionsaufsatz umfasst.

Bei dem hier angegebenen Kopplungselement ist vorgesehen, dass das wenigstens eine Grundelement eine Längsachse, eine Zentriereinrichtung, eine Stirnseite und eine an der Stirnseite ausgebildete Drehmomentaufnahmeeinrichtung umfasst, die zur Aufnahme eines von einem Gegenelement auf das Grundelement bezüglich der Längsachse ausgeübten Drehmomentes eine Mehrzahl von ausgehend von der Stirnseite ausgebildeten, in Achsumlaufrichtung mit Abstand voneinander verteilt angeordneten Auflagerelementen umfasst. Die Auflagerelemente weisen in einer Ansicht auf die Stirnseite jeweils eine Längsseite oder eine Schmalseite auf, wobei die Längsseite in ihrem Verlauf eine größere Länge aufweist als die Schmalseite. Die Schmalseite begrenzt eine sich parallel zur Längsachse und im Wesentlichen senkrecht zur Achsumlaufrichtung erstreckende Auflagerfläche, die zur Aufnahme eines in einem ersten Drehsinn wirksamen Drehmomentes von dem Gegenelement beaufschlagbar ist. Die Aufnahme eines von dem Gegenelement auf das Grundelement ausgeübten Drehmomentes erfolgt also über die Auflagerelemente, die ausgehend von der Stirnseite des Grundelementes ausgebildet sind und insbesondere eine Verkronung bilden. Dabei erfolgt die Krafteinleitung über Auflagerflächen der Auflagerelemente, die jeweils von der kürzeren Schmalseite der Auflagerelemente begrenzt sind. Durch die in ihrem Verlauf jeweils eine größere Länge als die Schmalseiten aufweisenden Längsseiten der Auflagerelemente wird den einwirkenden Kräften ein relativ hoher Widerstand entgegengesetzt, so dass für ein Abscheren der Auflagerelemente vergleichsweise hohe Kräfte wirksam sein müssen und folglich die Auflagerelemente in der Lage sind, hohe Drehmomente aufzunehmen.

Insbesondere ist der Verlauf der Längsseiten jeweils derart gewählt, dass die Längsseiten zumindest teilweise und insbesondere in einem sich unmittelbar an die jeweilige Auflagerfläche anschließenden Abschnitt zumindest näherungsweise in Verlängerung der bei Aufnahme eines Drehmomentes an der Auflagerfläche angreifenden Kraft verlaufen. Auf diese Weise steht die Längserstreckung der Auflagerelemente zur Verfügung, um den angreifenden Kräften einen relativ hohen Widerstand entgegen zu setzen und eine relativ hohe Scherfestigkeit der Auflagerelemente zu erreichen. Diese Ausgestaltung der Auflagerelemente ermöglicht des Weiteren eine bezüglich der Scherfestigkeit der Auflagerelemente optimale Ausnutzung der an der Stirnseite zur Verfügung stehenden Fläche. Von Bedeutung ist dies insbesondere dann, wenn aufgrund anderer Randbedingungen der Querschnitt des Grundelementes und damit dessen Stirnseite möglichst klein sein soll und zusätzlich die Zentriereinrichtung ebenfalls an der Stirnseite ausgebildet ist. Durch die Erstreckung der Auflagerflächen im Wesentlichen senkrecht zur Achsumlaufrichtung ergibt sich, dass die wirksamen Kräfte im Wesentlichen senkrecht in die Auflagerflächen eingeleitet werden, was sich hinsichtlich der Scherfestigkeit der Auflagerelemente ebenfalls günstig auswirkt.

Unter einer Erstreckung, die im Wesentlichen senkrecht zur Achsumlaufrichtung erfolgt, ist hier ein relativ großer Spielraum zu verstehen. Neigungen der Auflagerflächen gegenüber der Achsumlaufrichtung von bis 45°, insbesondere von bis zu 30°, bilden in dem hier vorgeschlagenen Sinne einen Verlauf im Wesentlichen senkrecht zur Achsumlaufrichtung.

In einer Ausführungsform liegen die Auflagerflächen jeweils zumindest näherungsweise in einer die Längsachse enthaltenden Ebene.

In einer weiteren Ausführungsform liegen die Längsseiten auf einem Vieleck, insbesondere einem Quadrat oder Rechteck.

In einer weiteren Ausführungsform liegen die Schmalseiten jeweils im Wesentlichen in der Mitte der jeweiligen Seite des Vielecks. Hierbei ist eine günstige Krafteinleitung in die Auflagerflächen insbesondere dann gewährleistet, wenn das Vieleck regelmäßig ist und die Längsachse des Grundelementes durch den Mittelpunkt des Vielecks verläuft.

In einer weiteren Ausführungsform liegen die Längsseiten, in Achsumlaufrichtung gesehen, in jedem zweiten Eckbereich. Dabei ist insbesondere vorgesehen, dass die Längsseiten V-förmig oder L-förmig ausgebildet sind, mit anderen Worten sich also "über Eck" erstrecken.

In spezifischen Anwendungen ist eine Drehmomentaufnahme nur in einem ersten Drehsinn von Bedeutung und im entgegengesetzten Drehsinn müssen entweder keine oder deutlich kleinere Drehmomente aufgenommen werden, so dass die Scherfestigkeit der Auflagerelemente bezüglich des entgegengesetzten Drehsinns von untergeordneter Bedeutung ist. Für einen solchen Fall schlägt eine weitere Ausführungsform vor, dass die Drehmomentaufnahmeeinrichtung derart drehsinnsensitiv ausgebildet ist, dass ein aufnehmbares Drehmoment bei in dem ersten Drehsinn beaufschlagten Auflagerelementen größer ist als bei in umgekehrtem Drehsinn beaufschlagten Auflagerelementen.

In einem Beispiel für eine derartige drehsinnsensitive Ausgestaltung sind drei Auflagerelemente vorgesehen, deren Längsachsen jeweils auf einer Seite eines Quadrats liegen, wobei von den Längsseiten begrenzte, im Wesentlichen senkrecht zur Längsachse verlaufende Stirnflächen der Auflagerelemente jeweils im Wesentlichen eine Rechteckform aufweisen und sich jeweils im Wesentlichen von der Mitte der jeweiligen Seite bis in einen Eckbereich des Quadrats erstrecken, in den sich keine der anderen Stirnflächen erstreckt.

Das hier angegebene Gegenelement, das zu Verwendung mit einem Kopplungselement, wie es vorstehend hinsichtlich einiger Aspekte erläutert wurde, vorgesehen ist, umfasst eine Längsachse, eine Zentriereinrichtung, die im gekoppelten Zustand mit der Zentriereinrichtung des Grundelements zusammenwirkt, derart, dass die Längsachse des Grundelementes und die Längsachse des Gegenelementes parallel zueinander verlaufen und insbesondere zusammenfallen, eine Stirnseite und eine an der Stirnseite ausgebildete, im gekoppelten Zustand mit der Drehmomentaufnahmeeinrichtung des Grundelementes zusammenwirkende Gegeneinrichtung. Die Gegeneinrichtung umfasst ausgehend von der Stirnseite ausgebildete Eingriffselemente, die im gekoppelten Zustand zwischen die Auflagerelemente des Grundelementes greifen.

In einer Ausführungsform des Gegenelementes sind dessen Eingriffselemente derart bemessen, dass sie im gekoppelten Zustand spielfrei an den von den Schmalseiten begrenzten Auflagerflächen der Auflagerelemente des Grundelementes anliegen. Ein von dem Gegenelement ausgeübtes, in dem ersten Drehsinn wirksames Drehmoment kann also, bezüglich der Achsumlaufrichtung gesehen, ohne Relativbewegung zwischen dem Grundelement und dem Gegenelement aufgenommen werden. Insbesondere ist durch die Spielfreiheit gewährleistet, dass alle Auflagerflächen gleichzeitig beaufschlagt werden, wodurch eine Belastung eines einzigen Auflagerelementes alleine vermieden wird.

In einer weiteren Ausgestaltung sind die Eingriffselemente des Gegenelementes derart bemessen, dass sie im gekoppelten Zustand bezüglich von den Längsseiten begrenzter Seitenflächen der Auflagerelemente des Grundelementes mit Spiel angeordnet sind. Eine solche Ausgestaltung ist insbesondere für solche Anwendungsfälle geeignet, bei denen - wie vorstehend bereits erwähnt - eine Drehmomentaufnahme nur bezüglich eines ersten Drehsinnes von Bedeutung ist. Indem bezüglich des entgegengesetzten Drehsinns zwischen den Auflagerelementen und den Eingriffselementen Spiel zugelassen wird, lässt sich die Herstellung des Grundelementes und des Gegenelementes aufgrund der erlaubten Toleranz vereinfachen.

In einer weiteren Ausgestaltung sind die mit den Auflagerelementen versehene Stirnseite des Grundelementes und die mit den Eingriffselementen versehene Stirnseite des Gegenelementes im Wesentlichen komplementär zueinander ausgebildet.

Bei drehsinnsensitiver Ausgestaltung der Drehmomentaufnahmeeinrichtung des Grundelementes in dem vorstehend erläuterten Sinne und diesbezüglich komplementärer Ausgestaltung des Gegenelementes ist also auch das Gegenelement in diesem Sinne drehsinnsensitiv ausgebildet.

In einer alternativen Ausführungsform ist das Gegenelement nicht komplementär, sondern insbesondere drehsinnneutral ausgebildet, so dass ein maximal von den Auflagerelementen des Grundelementes aufnehmbares Drehmoment von dessen Drehsinn unabhängig ist.

Hierbei ist in einer möglichen Ausführung vorgesehen, dass die Eingriffselemente des Gegenelementes nicht mit den von den Schmalseiten begrenzten Auflagerflächen der Auflagerelemente des Grundelementes, sondern mit den von den Längsseiten begrenzten Seitenflächen der Auflagerelemente zusammenwirken.

Es gibt Fälle, in denen - beispielsweise an einem einzigen Instrument - nicht nur ein Paar von Elementen, sondern beispielsweise zwei Paare gekoppelt werden müssen. Ein Beispiel für einen solchen Anwendungsfall sind Distraktionsinstrumente, die zwei Arme aufweisen, deren arbeitsseitige, distale Enden mit bestimmten Funktionsaufsätzen gekoppelt werden sollen. Die Arme des Distraktionsinstrumentes werden während der Operation auseinander bewegt, wobei auf die dabei voneinander weg bewegten Funktionsaufsätze im Wesentlichen entgegensetzt gerichtete Kräfte einwirken können. Wird zur Kopplung der Funktionsaufsätze ein Kopplungssystem der hier angegebenen Art eingesetzt und verlaufen die an den Funktionsaufsätzen angreifenden Kräfte anwendungsbedingt nicht durch die Längsachsen der Grundelemente oder Gegenelemente des Kopplungssystems, so werden von den Funktionsaufsätzen Drehmomente auf die Arme des Distraktionsinstrumentes ausgeübt. Diese Drehmomente müssen von den Kopplungsstellen, die jeweils von einem Grundelement und einem Gegenelement gebildet werden, aufgenommen werden, wobei bedingt durch die Konstruktion der Funktionsaufsätze der Fall auftreten kann, dass die von den Funktionsaufsätzen ausgeübten Drehmomente einen entgegengesetzten Drehsinn aufweisen. Für einen solchen Fall sieht eine Ausführungsform des hier angegebenen Kopplungssystems vor, dass zwei Grundelemente vorgesehen sind, die jeweils zusammen mit einem Gegenelement eine Kopplungsstelle bilden, wobei die beiden Kopplungsstellen derart gegensinnig oder spiegelbildlich ausgebildet sind, dass bei im gleichen Drehsinn belasteten Kopplungsstellen die aufnehmbaren maximalen Drehmomente entgegengesetzt gerichtet sind.

Vorstehend wurde bereits erwähnt, dass mit einem Grundelement koppelbare Gegenelemente unterschiedlich ausgestaltet sein können, wobei insbesondere sowohl drehsinnsensitive als auch drehsinnneutrale Gegenelemente vorgesehen werden können. Dementsprechend sieht eine Ausführungsform des Kopplungssystems vor, dass ein Satz von unterschiedlich ausgeführten Gegenelementen vorgesehen ist, wobei zumindest ein Gegenelement eine drehsinnsensitive Gegeneinrichtung aufweist, derart, dass ein aufnehmbares Drehmoment bei in dem ersten Drehsinn beaufschlagten Auflagerelementen größer ist als bei in umgekehrtem Drehsinn beaufschlagten Auflagerelementen, und wobei zumindest ein Gegenelement eine drehsinnneutrale Gegeneinrichtung aufweist, derart, dass ein maximales aufnehmbares Drehmoment im Wesentlichen drehsinnunabhängig ist. Eine derartige Ausgestaltung ist beispielsweise für solche Instrumentensysteme geeignet, bei denen ein Manipulationsinstrument sowohl mit Funktionsaufsätzen, über die wesentliche Drehmomente auf das Manipulationsinstrument ausgeübt werden, als auch mit Funktionsaufsätzen koppelbar sein soll, bezüglich welcher die Aufnahme von Drehmomenten keine oder nur eine untergeordnete Rolle spielt. Hier kann dann das Manipulationsinstrument mit einem drehsinnsensitiven Grundelement versehen sein, wohingegen die ein Drehmoment ausübenden Funktionsaufsätze mit einem entsprechend drehsinnsensitiven Gegenelement und die anderen Funktionsaufsätze mit einem drehsinnneutralen Gegenelement versehen werden.

Alle sowohl in den Ansprüchen als auch in der Beschreibung und in den Zeichnungen in Verbindung mit den hier angegebenen Gegenständen ggf. verwendeten und gemäß der fachüblichen Konvention gewählten Ausrichtungs-, Positionierungs-, Orientierungs- und Richtungsangaben, die insbesondere anatomische Achsen, Ebenen, Richtungen im Raum und Bewegungsrichtungen betreffen, sind dem Fachmann bekannt und beziehen sich ggf. auf den implantierten Zustand.

Nachfolgend wird die Erfindung anhand von in der Zeichnung illustrierten Ausführungsbeispielen näher erläutert. Dabei sollen die Ausführungsbeispiele und die Zeichnungen nur instruktiv verstanden werden und nicht zur Einschränkung der in den Ansprüchen beschriebenen Gegenstände dienen. Die Darstellungen in der Zeichnung sind vereinfacht; für das Verständnis der Erfindung nicht notwendige Einzelheiten sind weggelassen worden.

In der Zeichnung zeigt:
- Fig. 1a: eine Ausführungsform eines Grundelementes der hier angegebenen Art,
- Fig. 1b: eine Ausführungsform eines Gegenelementes der hier angegebenen Art, das mit dem Grundelement gemäß Fig. 1a koppelbar ist,
- Fig. 2a: das Grundelement von Fig. 1a und das Gegenelement von Fig. 1b einander gegenüberliegend,
- Fig. 2b: eine Ansicht auf die Stirnseite des Gegenelementes von Fig. 1b,
- Fig. 2c: eine Ansicht auf die Stirnseite des Grundelementes von Fig. 1a,
- Fig. 2d: eine Ansicht auf die Stirnseite eines Gegenelementes gemäß einer weiteren Ausführungsform,
- Fig. 2e: eine Ansicht auf die Stirnseite eines Grundelementes gemäß einer weiteren Ausführungsform,
- Fig. 3a: eine Ausführungsform eines mit einem Gegenelement der hier angegebenen Art versehenen caudalen Funktionsaufsatzes in Form eines Plattenhalters,
- Fig. 3b: eine andere Ansicht des Funktionsaufsatzes von Fig. 3a,
- Fig. 3c: eine Ansicht auf die Stirnseiten zweier gegensinnig ausgebildeter Funktionsaufsätze,
- Fig. 4: das Grundelement und das Gegenelement von Fig. 2a in unterschiedlichen Relativanordnungen, und
- Fig. 5: die Relativanordnungen von Fig. 4 mit teilweise geschnittenem Grundelement und Gegenelement.

Die Ausführungsbeispiele dienen dem besseren Verständnis der Erfindung und sollen nicht zu einer Einschränkung der in den Ansprüchen angegebenen Erfindung herangezogen werden.

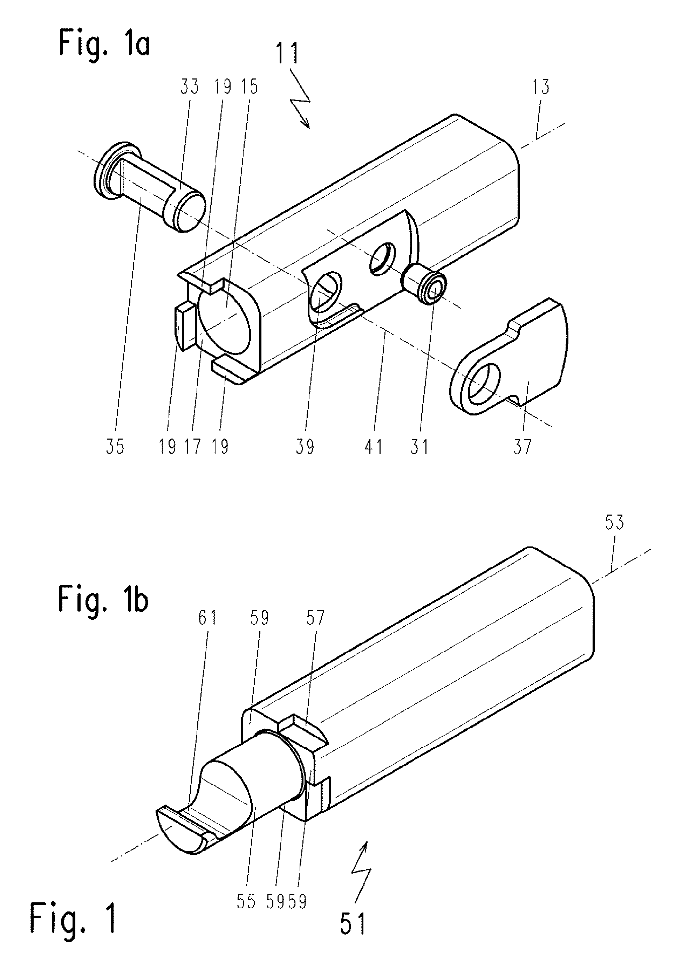

Fig. 1a zeigt ein Grundelement 11 und Fig. 1b zeigt ein Gegenelement 51, die hier jeweils als Stab mit quadratischem Querschnitt und abgerundeten Längskanten ausgebildet sind, wobei das Grundelement 11 eine Längsachse 13 und das Gegenelement 51 eine Längsachse 53 aufweist. Im Querschnitt gesehen fallen die Längsachsen 13, 53 jeweils mit dem Mittelpunkt des Quadrats zusammen, d.h. die Längsachsen 13, 53 sind insofern Mittelachsen der Elemente 11, 51. Ausgehend von einer senkrecht zur Mittelachse 13 verlaufenden Stirnseite 17 des Grundelementes 11 ist eine Aufnahme 15 in Form einer zentrischen Bohrung mit kreisförmigem Querschnitt im Grundelement 11 ausgebildet. Mit dieser Aufnahmebohrung 15 korrespondiert ein ausgehend von der senkrecht zur Mittelachse 53 verlaufenden Stirnseite 57 des Gegenelementes 51 ausgebildeter zentrischer Zapfen 55, der an seinem freien Ende hakenförmig ausgebildet ist und eine Ausnehmung 61 mit teilzylindrischem Querschnitt aufweist. Das Grundelement 11 und das Gegenelement 51 sind miteinander koppelbar, indem sie mit einander zugewandten Stirnseiten 17, 57 zusammen gesteckt oder zusammen geschoben werden. Die Aufnahmebohrung 15 des Grundelementes 11 und der in die Aufnahmebohrung 15 einführbare Zapfen 55 des Gegenelementes 51 dienen hierbei auch zur Zentrierung derart, dass im gekoppelten Zustand die beiden Mittelachsen 13, 53 zusammenfallen. Die Querschnitte des Grundelementes 11 und des Gegenelementes 51 sind gleich groß. Damit im gekoppelten Zustand ein von dem Gegenelement 51 bezüglich der Mittelachse 53 ausgeübtes Drehmoment von dem Grundelement 11 aufgenommen werden kann, ist das Grundelement 11 mit einer Drehmomentaufnahmeeinrichtung versehen, die von der Stirnseite 17 abstehende Auflagerelemente 19 umfasst. Kopplungsseitig ist das Grundelement 11 also auf diese Weise kronenartig oder zinnenartig ausgebildet. Eine mit der Drehmomentaufnahmeeinrichtung des Grundelementes 11 zusammenwirkende Gegeneinrichtung des Gegenelementes 51 ist im Wesentlichen komplementär zu der Drehmomentaufnahmeeinrichtung ausgebildet und umfasst von der Stirnseite 57 ausgehende Eingriffselemente 59, die im gekoppelten Zustand in die - bezüglich der Achsumlaufrichtung gesehen - zwischen den Auflagerelementen 19 des Grundelementes 11 vorhandenen Zwischenräume eingreifen.

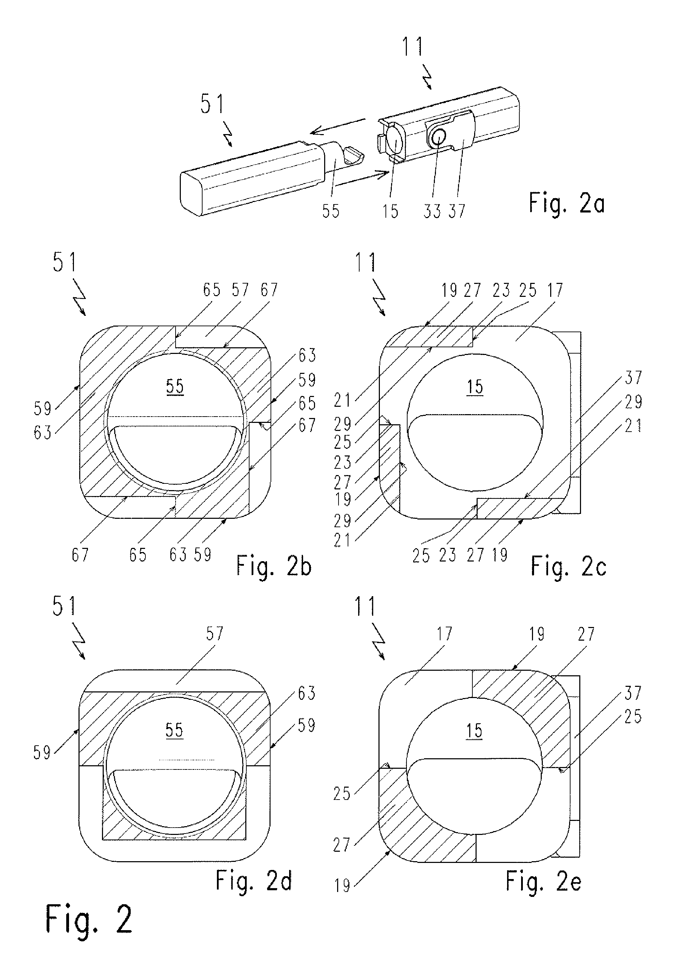

Auf die Ausgestaltung, Anordnung und das Zusammenwirken der Auflagerelemente 19 des Grundelementes 11 und der Eingriffselemente 59 des Gegenelementes 51 wird nachstehend in Verbindung mit den Fig. 2a bis 2e näher eingegangen. Das die erwähnte Ausnehmung 61 aufweisende hakenförmige freie Ende des Zentrierzapfens 55 des Gegenelementes 51 ist Bestandteil einer Verriegelungseinrichtung, die dazu dient, den gekoppelten Zustand zwischen Grundelement 11 und Gegenelement 51 in axialer Richtung zu sichern. Am Grundelement 11 umfasst die Verriegelungseinrichtung ein Verriegelungselement 33, das im zusammengesetzten Zustand in einer senkrecht zur Mittelachse 13 des Grundelementes 11 verlaufenden Querbohrung 39 um seine eigene Längsachse 41 drehbar angeordnet ist. Mittels eines für den Benutzer zugänglichen Betätigungshebels 37, der drehfest mit dem Verriegelungselement 33 verbunden ist, kann das Verriegelungselement 33 zwischen einer Freigabestellung und einer Blockierstellung verdreht werden. Ein federbelastetes Druckstück 31 dient zur Sicherung des Betätigungshebels 37 in der Blockierstellung, um ein unbeabsichtigtes Verdrehen des Verriegelungselementes 33 in dessen Freigabestellung zu verhindern.

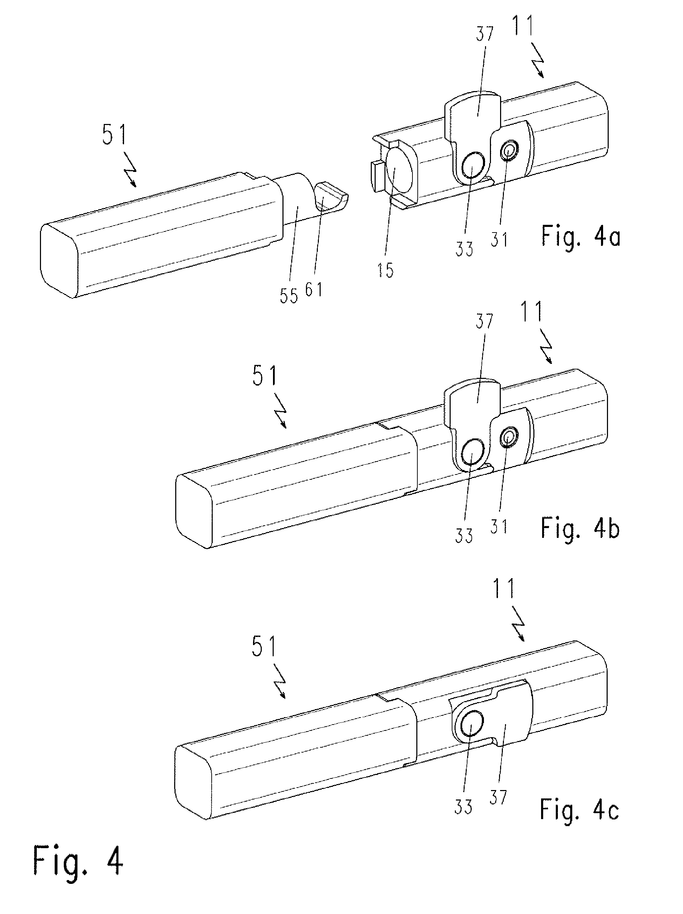

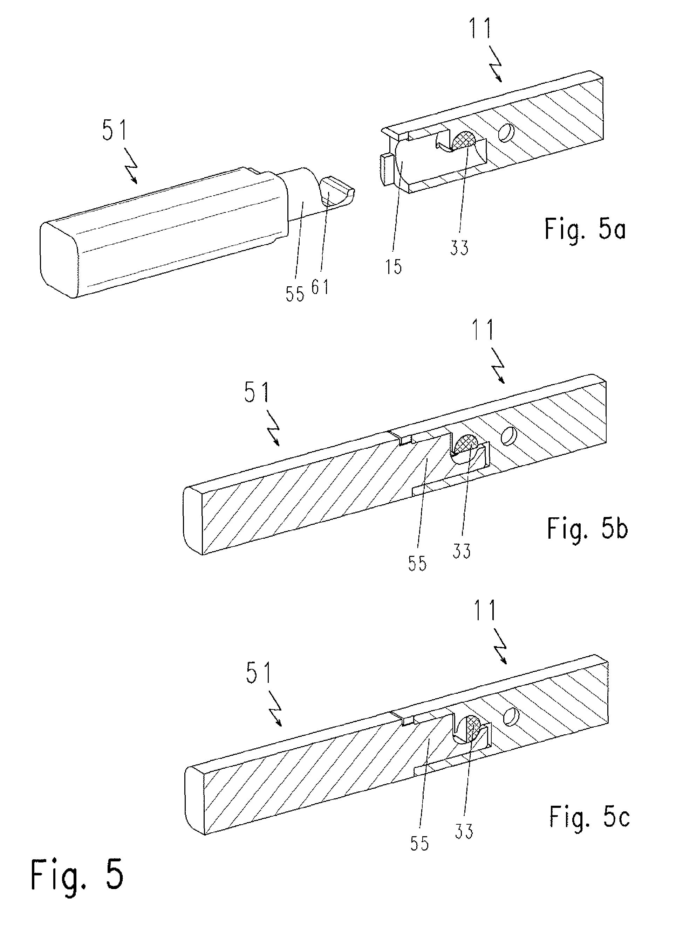

Auf die Funktionsweise dieser Verriegelungseinrichtung wird nachstehend in Verbindung mit Fig. 4 und 5 näher eingegangen.

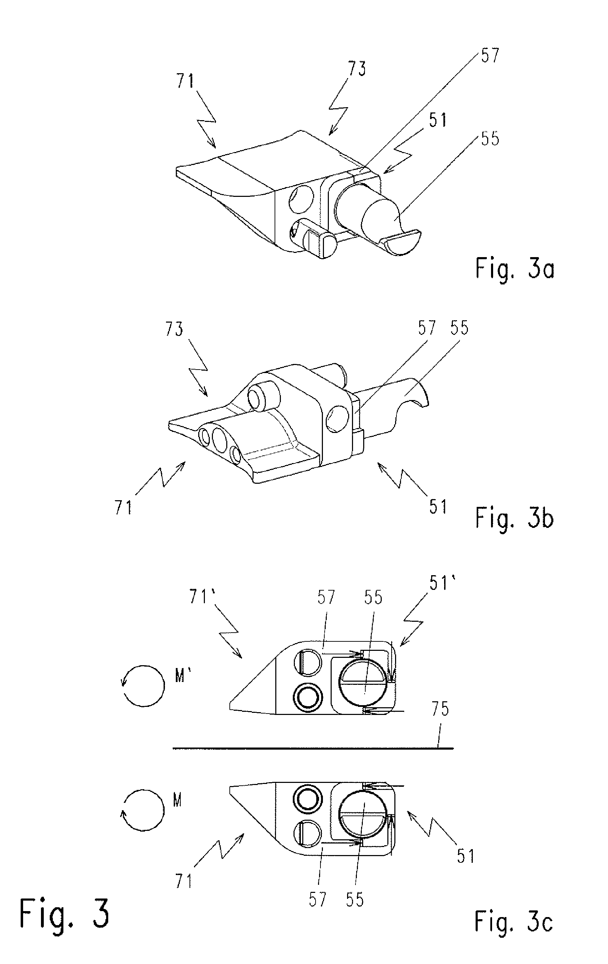

In Fig. 1a und Fig. 1b sind das Grundelement 11 und das Gegenelement 51 insofern vereinfacht darstellt, als in einer konkreten Ausführungsform sowohl das Grundelement 11 als auch das Gegenelement 51 Bestandteile von Vorrichtungen sind, die miteinander lösbar gekoppelt werden sollen. In einem möglichen Ausführungsbeispiel umfasst ein für Bandscheibenoperationen vorgesehenes Instrumentensystem ein Manipulationsinstrument in Form eines Distraktionsinstrumentes, das zwei Arme aufweist, an deren freien Enden jeweils ein Grundelement 11 ausgebildet ist. In diesem Ausführungsbeispiel umfasst das Instrumentensystem weiter verschiedene Funktionsaufsätze, die jeweils einen für eine jeweilige Applikation speziell ausgebildeten Funktionsabschnitt umfassen, an dem ein Gegenelement 51 ausgebildet ist. Zur lösbaren Anbringung der Funktionsaufsätze an dem Distraktionselement sind jeweils dessen Grundelemente 11 mit den Gegenelementen 51 der Funktionsaufsätze koppelbar. Hierauf und auf eine Besonderheit, die hierbei vorgesehen sein kann, wird nachstehend in Verbindung mit den Fig. 3a bis 3c näher eingegangen. Der Vollständigkeit halber sei erwähnt, dass das Instrumentensystem eine Mehrzahl von verschiedenen Instrumenten aufweisen kann, die jeweils ein oder mehrere Grundelemente entsprechend Fig. 1a aufweisen können, um die verschiedenartigsten, jeweils mit einem Gegenelement entsprechend Fig. 1b versehenen Funktionsaufsätze, die ebenfalls zum Instrumentensystem gehören, lösbar koppeln zu können. Ebenfalls der Vollständigkeit halber sei erwähnt, dass teilweise oder durchgehend auch die umgekehrte Anordnung möglich ist, wonach die Gegenelemente entsprechend Fig. 1b an den Manipulationsinstrumenten und die Grundelemente 11 entsprechend Fig. 1a an den Funktionsaufsätzen ausgebildet sind.

Fig. 2a zeigt das Grundelement 11 und das Gegenelement 51 einander gegenüberliegend mit einander zugewandten Stirnseiten und zusammenfallenden Mittelachsen angeordnet. Fig. 2b zeigt eine Ansicht auf die Stirnseite 57 des Gegenelementes 51, während in Fig. 2c eine Ansicht auf die Stirnseite 17 des Grundelementes 11 dargestellt ist. Die Stirnseiten 17, 57 verlaufen jeweils senkrecht zu der jeweiligen Mittelachse. Die Stirnflächen 63 der Eingriffselemente 59 des Gegenelementes 51 und die Stirnflächen 27 der Auflagerelemente 19 des Grundelementes 11 verlaufen jeweils ebenfalls senkrecht zu der jeweiligen Mittelachse und sind hier lediglich zur besseren Unterscheidung von der jeweiligen Stirnseite 17, 57 schraffiert dargestellt. Die kronenförmige Drehmomentaufnahmeeinrichtung des Grundelementes umfasst drei flachquaderförmige Auflagerelemente 19. Die Abweichung von einer exakten Flachquaderform ist durch die erwähnte Abrundung der Längskanten des Grundelementes 11 bedingt. In der Ansicht auf die Stirnseite 17 gemäß Fig. 2c weisen die Stirnflächen 27 der Auflagerelemente 19 folglich im Wesentlichen eine Rechteckform auf. Die Auflagerelemente 19 sind identisch ausgebildet und in Achsumlaufrichtung mit Abstand voneinander verteilt angeordnet, derart, dass auf drei der vier Seiten des quadratischen Querschnitts jeweils eine der im Wesentlichen rechteckigen Stirnflächen 27 liegt. Die Begrenzung der Stirnflächen 27 erfolgt jeweils zum einen durch die Außenkontur des Grundelementes 11 und zum anderen durch eine Längsseite 21 sowie eine Schmalseite 23. In dem dargestellten Ausführungsbeispiel sind die Längsseiten 21 jeweils um ein Vielfaches länger als die Schmalseiten 23. Die Längsseiten 21 sind hier jeweils ungefähr viermal so lang wie die Schmalseiten 23. Andere Längenverhältnisse können je nach konkreter Ausgestaltung des Grundelementes 11 vorgesehen sein. Die Längsseiten 21 begrenzen jeweils eine Seitenfläche 29 des Auflagerelementes 19, während die Schmalseiten 23 jeweils eine Auflagerfläche 25 des Auflagerelementes 19 begrenzen. Die Seitenflächen 29 und die Auflagerflächen 25 verlaufen jeweils parallel zur Längsachse des Grundelementes 11, wobei die Größe der Seitenflächen 29 und der Auflagerflächen 25 bei fester Länge der Längsseiten 21 und Schmalseiten 23 durch die axiale Länge der Auflagerelemente 19 bestimmt ist. In dem hier dargestellten Ausführungsbeispiel liegt die axiale Länge der Auflagerelemente 19 jeweils zwischen der Länge einer Schmalseite 23 und der Länge einer Längsseite 21. Die axiale Länge der Auflagerelemente 19 kann in Abhängigkeit von der jeweiligen Applikation variieren. Die rechteckigen Stirnflächen 27 der Auflagerelemente 19 erstrecken sich jeweils von der Mitte der jeweiligen Quadratseite bis in einen Eckbereich des Quadrats, wobei bezogen auf die Achsumlaufrichtung die Auflagerelemente 19 gleichsinnig orientiert sind, d.h. die rechteckigen Stirnflächen 27 erstrecken sich jeweils bis in einen Eckbereich, in den sich keine der anderen rechteckigen Stirnflächen 27 erstreckt. Die von den Schmalseiten 23 begrenzten Auflagerflächen 25 der Auflagerelemente 19 liegen jeweils etwa in einer die Mittelachse 13 des Grundelementes 11 enthaltenden Ebene und verlaufen somit etwa senkrecht zur Achsumlaufrichtung. Wie man einem Vergleich von Fig. 2c und Fig. 2b entnimmt, ist die von den Eingriffselementen 59 gebildete Gegeneinrichtung des Gegenelementes 51 komplementär zu der von den Auflagerelementen 19 gebildeten Drehmomentaufnahmeeinrichtung des Grundelementes 11 ausgebildet. Im gekoppelten Zustand greifen also die Eingriffselemente 59 in die bezogen auf die Achsumlaufrichtung zwischen den Auflagerelementen 19 vorhandenen Zwischenräume ein. Dabei sind die Auflagerelemente 19 und die Eingriffselemente 59 mit drehsinnabhängigen Toleranzwerten derart gefertigt, dass im gekoppelten Zustand die Eingriffselemente 59 mit Gegenflächen 65 spielfrei an den Auflagerflächen 25 der Auflagerelemente 19 anliegen, wohingegen zwischen den Seitenflächen 29 der Auflagerelemente 19 und entsprechenden Gegenflächen 67 der Eingriffselemente 59 Spiel vorhanden ist. Diese bezüglich der Achsumlaufrichtung vorgesehene Toleranzasymmetrie ermöglicht bei in fertigungstechnischer Hinsicht einfacher Herstellbarkeit extrem geringe Toleranzen in einem ersten Drehsinn, der sich dadurch auszeichnet, dass alle Auflagerflächen 25 der Auflagerelemente 19 des Grundelementes 11 von den entsprechenden Gegenflächen 65 der Eingriffselemente 59 des Gegenelementes 51 gleichzeitig beaufschlagt werden, wenn von dem Gegenelement 51 ein bezüglich der Mittelachsen in dem ersten Drehsinn wirksames Drehmoment auf das Grundelement 11 ausgeübt wird. Diese drehsinnsensitive Ausgestaltung sowohl der Drehmomentaufnahmeeinrichtung des Grundelementes 11 als auch der im Wesentlichen komplementären Gegeneinrichtung des Gegenelementes 51 ist für solche Applikationen besonders geeignet, bei denen die aufzunehmenden maximalen Drehmomente in dem ersten Drehsinn wirksam sind und in dem entgegengesetzten Drehsinn entweder keine Drehmomente oder wesentlich kleinere Drehmomente aufzunehmen sind. Es hat sich gezeigt, dass die anhand der Fig. 2b und 2c beschriebene Positionierung und Formgebung der Auflagerelemente 19 die Aufnahme von - bezogen auf die an der Stirnseite 17 zur Verfügung stehende Fläche - großen Drehmomenten gestatten. Die Auflagerelemente 19 weisen aufgrund dieser Ausgestaltung also eine hohe Scherfestigkeit auf. Dies ist insbesondere dann von Vorteil, wenn - neben anderen Anforderungen - mit den Grundelementen 11 versehene Operationsinstrumente und mit den Gegenelementen 51 versehene Funktionsaufsätze für die Instrumente aus operationstechnischen Gründen einen möglichst kleinen Querschnitt aufweisen sollen und gleichzeitig ein bestimmter Anteil des Querschnitts für Zentriereinrichtungen - hier für die Aufnahmebohrung 15 im Grundelement 11 und für den Zentrierzapfen 55 am Gegenelement 51 - bereitgestellt werden muss. Das im gekoppelten Zustand gegebene spielfreie Zusammenwirken der Auflagerflächen 25 und der Gegenflächen 65 sorgt dafür, dass bei einem vom Gegenelement 51 ausgeübten Drehmoment alle drei Auflagerflächen 25 gleichzeitig beaufschlagt werden und somit das Drehmoment von allen drei Auflagerelementen 19 in gleichem Maße aufgenommen wird. Mit anderen Worten wird die Last auf alle drei Auflagerelemente 19 gleichmäßig verteilt. Es har sich gezeigt, dass für in der Praxis erwartete maximale Drehmomente die bei dem Ausführungsbeispiel vorgesehenen drei Auflagerelemente 19 ausreichend sind, auf ein viertes Auflagerelement an der in dem dargestellten Ausführungsbeispiel freien Seite des quadratischen Querschnitts also verzichtet werden kann. Die maschinelle Bearbeitung zur Herstellung der stirnseitigen Drehmomentaufnahmeeinrichtung wird hierdurch erheblich vereinfacht.

Fig. 2e zeigt eine andere Ausführungsform einer an einem Grundelement 11 stirnseitig ausgebildeten Drehmomentaufnahmeeinrichtung, die hier zwei Auflagerelemente 19 aufweist. Die Auflagerelemente 19 sind ausgehend von der Stirnseite 17 an Bereichen von einander gegenüberliegenden Quadranten der Stirnseite17 ausgebildet, die nach Einbringen der Zentrieraufnahme 15 verblieben sind. In einer Ansicht auf die Stirnseite 17 sind die Auflagerelemente 19 folglich im Wesentlichen V-förmig ausgebildet, erstrecken sich also jeweils "über Eck". Die vorstehend erläuterte Toleranzasymmetrie in Bezug auf eine nicht dargestellte Gegeneinrichtung eines Gegenelementes ist auch bei diesem Ausführungsbeispiel vorgesehen. Die beiden Auflagerflächen 25 werden folglich auch hier bei einem in dem ersten Drehsinn wirksamen Drehmoment gleichzeitig beaufschlagt. Das Ausführungsbeispiel der Fig. 2e zeichnet sich unter anderem durch eine besonders einfache Herstellbarkeit aus.

Die vorstehend erläuterte Drehsinnsensitivität ist für bestimmte Applikationen von Bedeutung, wie sie beispielhaft nachstehend in Verbindung mit Fig. 3a - 3c beschrieben werden. Um universell einsetzbare Grundelemente verwenden zu können, die sowohl für drehsinnsensitive Applikationen geeignet sind als auch mit solchen Gegenelementen gekoppelt werden können, bei denen Drehmomente per se oder hinsichtlich ihrer Größe allenfalls eine untergeordnete Rolle spielen, sind in einem Ausführungsbeispiel Gegenelemente 51 vorgesehen, wie sie beispielhaft in Fig. 2d gezeigt sind. Das Gegenelement 51 gemäß Fig. 2d ist insbesondere mit dem Ziel einer universellen Verwendbarkeit derart ausgebildet, dass es mit zwei unterschiedlich ausgebildeten Grundelementen 11 gekoppelt werden kann, die sich dadurch voneinander unterscheiden, dass ihre Drehmomentaufnahmeeinrichtungen gegensinnig oder spiegelbildlich ausgebildet sind. Was hierunter zu verstehen ist, wird nachstehend in Verbindung mit Fig. 3c erläutert.

Fig. 3a und Fig. 3b zeigen jeweils eine perspektivische Ansicht eines Beispiels für einen Funktionsaufsatz 71, der hier als ein Plattenhalter ausgebildet ist, welcher Bestandteil eines bei Bandscheibenoperationen einsetzbaren Instrumentensystems ist. Der Plattenhalter 71 umfasst einen Funktionsabschnitt 73, der mit einem Gegenelement 51 entsprechend Fig. 1b und Fig. 2b versehen ist. An seinem distalen Ende ist der Funktionsabschnitt 73 zur Halterung einer Implantatplatte ausgebildet, worauf hier nicht näher eingegangen werden soll. An der proximalen Seite des Funktionsabschnitts 73 sind eine mit einer Drehmomentaufnahmeeinrichtung eines Grundelementes koppelbare Gegeneinrichtung und ein Zentrierzapfen 55 ausgebildet. Das erwähnte Grundelement ist entsprechend Fig. 1a und Fig. 2c ausgebildet und am distalen Ende eines der beiden Arme eines als Distraktionsinstrument ausgebildeten Manipulationsinstrumentes angeordnet. Fig. 3a und Fig. 3b zeigen einen caudalen Plattenhalter, der in Fig. 3c in einer Ansicht auf die Stirnseite 57, also auf die proximale Seite, unterhalb einer Symmetrieebene 75 dargestellt ist. Oberhalb der Symmetrieebene 75 ist in einer entsprechenden Ansicht ein cranialer Funktionsaufsatz 71' dargestellt, der an seiner proximalen Seite als Gegenelement 51' ausgebildet ist. Der craniale Funktionsaufsatz 71' ist mit dem an dem anderen Arm des Distraktionsinstrumentes ausgebildeten Grundelement koppelbar. Das erwähnte Distraktionsinstrument ist Bestandteil eines Instrumentensystems zum Einsetzen eines Bandscheibenimplantats, das zusätzlich zu einer caudalen Implantatplatte und einer cranialen Implantatplatte einen im implantierten Zustand zwischen den beiden Implantatplatten angeordneten Einsatz oder Kern umfasst. Ein derartiges Bandscheibenimplantat ist unter dem Namen "Dynardi" der Anmelderin bekannt. Ein Operationsschritt zum Einsetzen dieses Bandscheibenimplantats beinhaltet das Einführen von an Plattenhaltern 71, 71' gehalterten Implantatplatten in den vorbereiteten Zwischenraum zwischen den betreffenden benachbarten Wirbelkörpern. Die Plattenhalter 71, 71' sind hierbei mit den freien Enden der beiden Arme des Distraktionsinstrumentes gekoppelt, wobei die nachstehend in Verbindung mit Fig. 4 und Fig. 5 beschriebene Verriegelungseinrichtung für eine axial feste Verbindung sorgt und die Drehmomentaufnahmeeinrichtungen der an den distalen Enden der Arme ausgebildeten Grundelemente jeweils mit der Gegeneinrichtung des betreffenden Plattenhaltes 71, 71' zusammenwirken. Um den erwähnten Einsatz oder Kern des Implantats zwischen die beiden Implantatplatten einführen zu können, erfolgt mit Hilfe des Distraktionsinstrumentes eine Distraktion der beiden Implantatplatten, d.h. über die beiden Plattenhalter 71, 71' werden die Implantatplatten gegen den Widerstand der Wirbelkörper, an denen die Implantatplatten caudal bzw. cranial anliegen, auseinandergedrückt und dabei im Wesentlichen senkrecht zu der in Fig. 3c dargestellten Symmetrieebene 75 bewegt. Konstruktionsbedingt erzeugen bei dieser Distraktion die über die Implantatplatten auf die Plattenhalter 71, 71' ausgeübten Kräfte jeweils ein Drehmoment M, M' bezüglich der Mittelachse des jeweiligen Zentrierzapfens 55. Wie aus Fig. 3c unmittelbar hervorgeht, sind diese Drehmomente M, M' entgegengesetzt gerichtet. In dieser Applikation sind daher bei den hier ein Paar bildenden Funktionsaufsätzen 71, 71' die Gegeneinrichtungen der Gegenelemente 51, 51' und die nicht dargestellten zugehörigen Drehmomentaufnahmeeinrichtungen der Grundelemente spiegelbildlich oder gegensinnig ausgebildet, um die bei der Distraktion wirksamen, entgegengesetzt gerichteten Drehmomente M, M' aufnehmen zu können. Mit den Pfeilen sind in Fig. 3c die Reaktionskräfte der Auflagerflächen 25 der Grundelemente 11 (Fig. 2c) angedeutet, die von den Gegenflächen 65 der Gegenelemente 51 (Fig. 2b) beaufschlagt werden.

Allgemein ausgedrückt sorgt also die erläuterte drehsinnsensitive Ausgestaltung der Mittel zur Drehmomentaufnahme einerseits für eine vergleichsweise hohe Scherfestigkeit der zusammenwirkenden stirnseitigen Verkronungen, also der Auflagerelemente 19 des Grundelementes und der Eingriffselemente 59 des Gegenelements. Andererseits hat die drehsinnsensitive Ausgestaltung zur Folge, dass bei bestimmten Anwendungen - wie der anhand von Fig. 3c beschriebenen Distraktion - die beiden Grundelemente und die beiden Gegenelemente gegensinnig oder spiegelbildlich zueinander ausgebildet sein müssen.

Fig. 4 und Fig. 5 veranschaulichen die Funktionsweise der eingangs bereits erwähnten Verriegelungseinrichtung, mit der im gekoppelten Zustand eine in axialer Richtung feste Verbindung zwischen dem Grundelement 11 und dem Gegenelement 51 herstellbar ist. Wie bereits in Verbindung mit Fig. 1a und Fig. 1b erwähnt, ist am Grundelement 11 ein Verriegelungselement 33 vorgesehen, das im Wesentlichen die Form eines mit einer Aussparung 35 versehenen Zylinders aufweist. Der im Bereich der Aussparung 35 verbliebene Teil des Zylinders ist durch Drehen des Verriegelungselementes 33 in Eingriff und außer Eingriff mit der am freien Ende des Zentrierzapfens 55 des Gegenelementes 51 ausgebildeten Ausnehmung 61 bringbar. In der oberen Darstellung der Fig. 4 und Fig. 5 ist die Verriegelungseinrichtung jeweils geöffnet. Das Verriegelungselement 33 befindet sich in einer Freigabestellung, in welcher der Zapfen 55 in die Aufnahmebohrung 15 des Grundelementes 11 an dem Verriegelungselement 33 vorbei eingeführt werden kann. Der vollständig eingeschobene, aber noch nicht verriegelte Zustand ist jeweils in der mittleren Darstellung der Fig. 4 und Fig. 5 gezeigt. Die Verriegelungseinrichtung wird geschlossen, indem ein Benutzer den Betätigungshebel 37 (Fig. 4) und damit das drehfest mit dem Betätigungshebel 37 verbundene Verriegelungselement 33 um 90° in die Blockierstellung dreht, die jeweils in der unteren Darstellung der Fig. 4 und Fig. 5 gezeigt ist. Der im Bereich der Aussparung 35 verbliebene Teil des Verriegelungselementes 33 befindet sich jetzt in Eingriff mit der am Zapfen 55 ausgebildeten Ausnehmung 61. Ein Herausziehen des Zapfens 55 aus dem Grundelement 11 ist jetzt nicht mehr möglich, d.h. das Grundelement 11 und das Gegenelement 51 sind axial fest miteinander verbunden. In dem hierdurch hergestellten gekoppelten Zustand befinden sich auch die Drehmomentaufnahmeeinrichtung des Grundelementes 11 und die in diesem Ausführungsbeispiel komplementär dazu ausgebildete Gegeneinrichtung des Gegenelementes 51 miteinander in Eingriff. Das in Fig. 4 dargestellte federbelastete Druckstück 31 wirkt mit dem sich in der geschlossenen Stellung befindenden Betätigungshebel 37 derart zusammen, dass vom Benutzer eine Mindestkraft aufgebracht werden muss, um gegen die Wirkung des Druckstücks 31 den Betätigungshebel 37 in die Freigabestellung zu drehen. Der gekoppelte Zustand ist hierdurch gesichert.

Im Lichte der hier gemachten Ausführungen eröffnen sich dem Fachmann weitere Ausführungsformen der in den Ansprüchen angegebenen Gegenstände, welche hier nicht abschließend dargestellt werden können.

### Bezugszeichenliste

- 11: Grundelement
- 13: Längsachse des Grundelementes
- 15: Zentriereinrichtung, Aufnahme
- 17: Stirnseite des Grundelementes
- 19: Auflagerelement
- 21: Längsseite des Auflagerelementes
- 23: Schmalseite des Auflagerelementes
- 25: Auflagerfläche des Auflagerelementes
- 27: Stirnfläche des Auflagerelementes
- 29: Seitenfläche des Auflagerelementes
- 31: Druckstück
- 33: Verriegelungselement
- 35: Aussparung
- 37: Betätigungshebel
- 39: Querbohrung
- 41: Längsachse des Verriegelungselementes
- 51, 51': Gegenelement
- 53: Längsachse des Gegenelementes
- 55: Zentriereinrichtung, Zapfen
- 57: Stirnseite des Gegenelementes
- 59: Eingriffselement des Gegenelementes
- 61: Ausnehmung
- 63: Stirnfläche des Eingriffselementes
- 65: Gegenfläche des Eingriffselementes
- 67: Gegenfläche des Eingriffselementes
- 71, 71': Funktionsaufsatz, Plattenhalter
- 73: Funktionsabschnitt
- 75: Symmetrieebene
- M, M': Drehmoment

## Patentansprüche

1. Kopplungselement, umfassend
wenigstens ein Grundelement (11) mit einer Längsachse (13), einer Zentriereinrichtung (15), einer Stirnseite (17) und einer an der Stirnseite (17) ausgebildeten Drehmomentaufnahmeeinrichtung zur Aufnahme eines von einem Gegenelement (51, 51') auf das Grundelement (11) bezüglich der Längsachse (13) ausgeübten Drehmomentes,
wobei die Drehmomentaufnahmeeinrichtung eine Mehrzahl von ausgehend von der Stirnseite (17) ausgebildeten, in Achsumlaufrichtung mit Abstand voneinander verteilt angeordneten Auflagerelementen (19) umfasst,
wobei die Auflagerelemente (19) in einer Ansicht auf die Stirnseite (17) jeweils eine Längsseite (21) und eine Schmalseite (23) aufweisen, wobei die Längsseite (21) in ihrem Verlauf eine größere Länge aufweist als die Schmalseite (23), und
wobei die Schmalseite (23) eine sich parallel zur Längsachse (13) und im Wesentlichen senkrecht zur Achsumlaufrichtung erstreckende Auflagerfläche (25) begrenzt, die zur Aufnahme eines in einem ersten Drehsinn wirksamen Drehmomentes von dem Gegenelement (51, 51') beaufschlagbar ist.

2. Kopplungselement nach Anspruch 1,
**dadurch gekennzeichnet,**
**dass** die Auflagerflächen (25) jeweils zumindest näherungsweise in einer die Längsachse (13) enthaltenden Ebene liegen.

3. Kopplungselement nach Anspruch 1 oder 2,
**dadurch gekennzeichnet,**
**dass** die Längsseiten (21) auf einem Vieleck, insbesondere einem Quadrat oder Rechteck, liegen.

4. Kopplungselement nach Anspruch 3,
**dadurch gekennzeichnet,**
**dass** die Schmalseiten (23) jeweils im Wesentlichen in der Mitte der jeweiligen Seite des Vielecks liegen.

5. Kopplungselement nach Anspruch 3 oder 4,
**dadurch gekennzeichnet,**
**dass** auf jeder Seite des Vielecks maximal eine der Längsseiten (21) ganz oder teilweise liegt, wobei insbesondere auf wenigstens einer der Seiten des Vielecks keine Längsseite liegt.

6. Kopplungselement nach einem der Ansprüche 3 bis 5,
**dadurch gekennzeichnet,**
**dass** die Längsseiten (21), in Achsumlaufrichtung gesehen, in jedem zweiten Eckbereich liegen und insbesondere V-förmig oder L-förmig ausgebildet sind.

7. Kopplungselement nach einem der vorhergehenden Ansprüche,
**dadurch gekennzeichnet,**
**dass** die Drehmomentaufnahmeeinrichtung derart drehsinnsensitiv ausgebildet ist, dass ein aufnehmbares Drehmoment bei in dem ersten Drehsinn beaufschlagten Auflagerelementen (19) größer ist als bei in umgekehrtem Drehsinn beaufschlagten Auflagerelementen (19).

8. Kopplungselement nach einem der vorhergehenden Ansprüche,
**dadurch gekennzeichnet,**
**dass** drei Auflagerelemente (19) vorgesehen sind, deren Längsseiten (21) jeweils auf einer Seite eines Quadrats liegen, wobei von den Längsseiten (21) begrenzte, im Wesentlichen senkrecht zur Längsachse (13) verlaufende Stirnflächen (27) jeweils im Wesentlichen eine Rechteckform aufweisen und sich jeweils im Wesentlichen von der Mitte der jeweiligen Seite bis in einen Eckbereich des Quadrats erstrecken, in den sich keine der anderen Stirnflächen (27) erstreckt.

9. Gegenelement zur Verwendung mit einem Kopplungselement nach einem der vorhergehenden Ansprüche, umfassend
eine Längsachse (53), eine Zentriereinrichtung (55), die im gekoppelten Zustand mit der Zentriereinrichtung (15) des Grundelementes (11) zusammenwirkt, derart, dass die Längsachse (13) des Grundelementes (11) und die Längsachse (53) des Gegenelementes (51, 51') parallel zueinander verlaufen und insbesondere zusammenfallen, eine Stirnseite (57) und eine an der Stirnseite (57) ausgebildete, im gekoppelten Zustand mit der Drehmomentaufnahmeeinrichtung des Grundelementes (11) zusammenwirkende Gegeneinrichtung,
wobei die Gegeneinrichtung ausgehend von der Stirnseite (57) ausgebildete Eingriffselemente (59) umfasst, die im gekoppelten Zustand zwischen die Auflagerelemente (19) des Grundelementes (11) greifen.

10. Gegenelement nach Anspruch 9,
**dadurch gekennzeichnet,**
**dass** die Eingriffselemente (59) des Gegenelementes (51, 51') derart bemessen sind, dass sie im gekoppelten Zustand spielfrei an den von den Schmalseiten (23) begrenzten Auflagerflächen (25) der Auflagerelemente (19) des Grundelementes (11) anliegen.

11. Gegenelement nach Anspruch 9 oder 10,
**dadurch gekennzeichnet,**
**dass** die Eingriffselemente (59) des Gegenelementes (51, 51') derart bemessen sind, dass sie im gekoppelten Zustand bezüglich von den Längsseiten (21) begrenzter Seitenflächen (29) der Auflagerelemente (19) des Grundelementes (11) mit Spiel angeordnet sind.

12. Gegenelement nach einem der Ansprüche 9 bis 11,
**dadurch gekennzeichnet,**
**dass** die mit den Auflagerelementen (19) versehene Stirnseite (17) des Grundelementes (11) und die mit den Eingriffselementen (59) versehene Stirnseite (57) des Gegenelementes (51) im Wesentlichen komplementär zueinander ausgebildet sind.

13. Kopplungssystem, umfassend
wenigstens ein Grundelement (11) nach einem der Ansprüche 1 bis 8 und wenigstens ein Gegenelement (51, 51') nach einem der Ansprüche 9 bis 12.

14. Kopplungssystem nach Anspruch 13,
**dadurch gekennzeichnet,**
**dass** zwei Grundelemente (11) vorgesehen sind, die jeweils zusammen mit einem Gegenelement (51, 51') eine Kopplungsstelle bilden, wobei die beiden Kopplungsstellen derart gegensinnig ausgebildet sind, dass bei im gleichen Drehsinn belasteten Kopplungsstellen die aufnehmbaren maximalen Drehmomente entgegengesetzt gerichtet sind.

15. Kopplungssystem nach Anspruch 13 oder 14,
**dadurch gekennzeichnet,**
**dass** ein Satz von unterschiedlich ausgeführten Gegenelementen (51) vorgesehen ist, wobei zumindest ein Gegenelement (51) eine drehsinnsensitive Gegeneinrichtung aufweist, derart, dass ein aufnehmbares Drehmoment bei in dem ersten Drehsinn beaufschlagten Auflagerelementen (19) größer ist als bei in umgekehrtem Drehsinn beaufschlagten Auflagerelementen (19), und
wobei zumindest ein Gegenelement (51) eine drehsinnneutrale Gegeneinrichtung aufweist, derart, dass ein maximales aufnehmbares Drehmoment im Wesentlichen drehsinnunabhängig ist.

16. Kopplungssystem nach einem der Ansprüche 13 bis 15,
**dadurch gekennzeichnet,**
**dass** das Kopplungssystem als Instrumentenschnittstelle zwischen wenigstens einem Manipulationsinstrument und zumindest einem mit dem Manipulationsinstrument koppelbaren Funktionsaufsatz 71, 71', insbesondere für Bandscheibenoperationen, ausgebildet ist.

17. Instrumentensystem, insbesondere für Bandscheibenoperationen, umfassend
wenigstens ein Manipulationsinstrument und
zumindest einen mit dem Manipulationsinstrument koppelbaren Funktionsaufsatz (71, 71'),
wobei zur Koppelung des Manipulationsinstrumentes mit dem Funktionsaufsatz (71, 71') ein Kopplungssystem nach einem der Ansprüche 13 bis 16 vorgesehen ist,
wobei insbesondere das Grundelement (11) an dem Manipulationsinstrument und das Gegenelement (51, 51') an dem Funktionsaufsatz (71, 71') ausgebildet ist.

18. Instrumentensystem nach Anspruch 17,
**dadurch gekennzeichnet,**
**dass** das Manipulationsinstrument ein Distraktionsinstrument ist, das zwei Arme aufweist, an deren Enden jeweils ein Grundelement (11) oder Gegenelement ausgebildet ist, wobei durch Betätigen des Distraktionsinstrumentes die Grundelemente (11) oder Gegenelemente im Wesentlichen in einer Richtung senkrecht zu ihren Längsachsen (13) voneinander weg bewegbar sind.

19. Instrumentensystem nach Anspruch 17 oder 18,
**dadurch gekennzeichnet,**
**dass** ein Satz von Funktionsaufsätzen (71) vorgesehen ist, die jeweils einerseits einen Funktionsabschnitt (73) und andererseits ein Grundelement oder ein Gegenelement (51) des Kopplungssystems umfassen, wobei sich die Funktionsaufsätze (71) hinsichtlich der Funktionsabschnitte (73) voneinander unterscheiden, und wobei die Grundelemente oder Gegenelemente (51) der Funktionsaufsätze (71) identisch ausgeführt sind.
